# EUROPÄISCHE PATENTSCHRIFT

(11) **EP 0 741 170 B1**
(45) Veröffentlichungstag und Bekanntmachung des Hinweises auf die Patenterteilung: **29.12.1999**
(21) Anmeldenummer: 96106345.0
(22) Anmeldetag: 23.04.1996
(51) Int. Cl.: C09C 1/00, C09C 3/06, C09D 7/12, C09D 11/00, C08K 9/02, C03C 4/02, C04B 33/14, A61K 7/00

(54) **Goniochromatische Glanzpigmente mit Aluminiumbeschichtung**
Brilliant goniochromatic pigments having an aluminium coating
Pigments brillants goniochromatiques à revêtement d'aluminium

(30) Priorität: 03.05.1995 DE 19516181
(43) Veröffentlichungstag der Anmeldung: 06.11.1996
(73) Patentinhaber: BASF AKTIENGESELLSCHAFT, 67056 Ludwigshafen (DE)
(72) Erfinder: Schmid, Raimund, Dr., 67435 Neustadt (DE); Mronga, Norbert, Dr., 69221 Dossenheim (DE); Adel, Jörg, Dr., 67071 Ludwigshafen (DE)

(56) Entgegenhaltungen:
- EP-A- 0 571 836
- EP-A- 0 668 329
- EP-A- 0 686 675
- EP-A- 0 708 155
- US-A- 3 438 796

## Beschreibung

Die vorliegende Erfindung betrifft neue goniochromatische Glanzpigmente auf der Basis von durch Mehrfachbeschichtung vollständig umhüllten plättchenförmigen metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer im wesentlichen aus Aluminium bestehenden, metallischen, für sichtbares Licht zumindest teilweise durchlässigen Schicht
sowie gewünschtenfalls zusätzlich
C) eine äußere Schicht, die im wesentlichen aus farblosem oder selektiv absorbierendem Metalloxid besteht und/oder phosphat-, chromat- und/oder vanadathaltig ist,
aufweisen.

Weiterhin betrifft die Erfindung Mischungen aus den Pigmenten (I) und mehrfach beschichteten silikatischen Plättchen (II), die
A') eine im wesentlichen aus farblosem oder selektiv absorbierendem Metalloxid bestehende Schicht,
B') eine im wesentlichen aus Aluminium bestehende, metallische, für sichtbares Licht zumindest teilweise durchlässige Schicht und gewünschtenfalls
C') eine äußere Schicht, die im wesentlichen aus farblosem oder selektiv absorbierendem Metalloxid besteht und/oder phosphat-, chromat- und/oder vanadathaltig ist,
aufweisen, als wesentlichen Komponenten.

Außerdem betrifft die Erfindung die Herstellung der goniochromatischen Glanzpigmente und ihre Verwendung zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Glanzpigmente auf Basis metallischer Substrate sind aufgrund ihres guten Deckvermögens auch für Automobillackierungen von besonderem Interesse.

Bislang werden hierfür vor allem eisenoxidbeschichtete Aluminiumpigmente, wie sie in der EP-A-33 457 beschrieben werden, eingesetzt, die im Glanzwinkel intensive, goldene bis rote Reflexionsfarben zeigen, bei steileren Betrachtungswinkeln jedoch unbunt wirken. Um Lackierungen mit einem sogenannten two-tone Effekt zu erzielen, werden diese Pigmente daher mit Buntpigmenten anderer Farbe gemischt.

Goniochromatische Pigmente, die einen winkelabhängigen Farbwechsel zwischen unterschiedlichen Interferenzfarben zeigen, also bereits ohne Zusatz weiterer Pigmente in Lackierungen oder Drucken einen two-tone Effekt bewirken, sind z.B. aus der US-A-3 438 796 bekannt, in der symmetrisch aufgebaute Pigmente beschrieben werden, die aus einem zentralen opaken Aluminiumfilm (60 nm dick) bestehen, der beidseitig jeweils mit einem dickeren SiO₂-Film (500 nm), einem transparenten Aluminiumfilm (20 nm) und einem dünneren SiO₂-Film (200 nm) beschichtet ist.

Diese Pigmente werden hergestellt, indem eine Substratfolie im Hochvakuum abwechselnd mit SiO₂ und Aluminium bedampft wird. Der auf diese Weise aufgedampfte mehrschichtige Film wird anschließend von der Folie entfernt, z.B. abgekratzt, und auf für Glanzpigmente typische Teilchengrößen (etwa 5-50 µm) zerkleinert.

Durch die Art der Herstellung bedingt, ist der zentrale Metallfilm dieser Pigmente nur an der Plättchenober- und -unterseite beschichtet und daher nur unvollständig vor Umwelteinflüssen oder dem Angriff von Chemikalien geschützt. Zudem ist das Herstellungsverfahren auch sehr aufwendig und kostspielig.

Aus den nicht vorveröffentlichten DE-A-44 05 492 und 44 19 173 sind goniochromatische Glanzpigmente bekannt, bei denen Aluminiumplättchen, die im Fall der DE-A-44 19 173 mit einer inneren ferromagnetischen Schicht belegt und daher magnetisierbar sind, zunächst naßchemisch durch hydrolytische Zersetzung siliciumorganischer Verbindungen mit Siliciumoxid und anschließend durch Gasphasenbeschichtung (chemical vapor deposition, CVD) mit metallischen Schichten belegt werden. Aus der nicht vorveröffentlichten DE-A-44 37 752 ist ein CVD-Verfahren bekannt, nach dem die SiO₂-Schicht auch durch Gasphasenzersetzung von Siliciumorganylen aufgebracht werden kann. Glanzpigmente, die Aluminiumbeschichtungen enthalten, werden jedoch nicht beschrieben.

Der Erfindung lag daher die Aufgabe zugrunde, goniochromatische Glanzpigmente ohne die genannten Nachteile und mit vorteilhaften Anwendungseigenschaften bereitzustellen.

Demgemäß wurden die eingangs definierten Glanzpigmente und ihre Mischungen mit mehrfach beschichteten silikatischen Plättchen gefunden.

Weiterhin wurde ein Verfahren zur Herstellung dieser Glanzpigmente gefunden, welches dadurch gekennzeichnet ist, daß man
die Schichten (A) durch Gasphasenzersetzung flüchtiger Siliciumorganyle mit Wasserdampf und/oder Sauerstoff oder durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind, und anschließende Trocknung,
die metallischen Schichten (B) durch Gasphasenzersetzung flüchtiger Aluminiumalkyle oder Trialkylaminaddukte von Aluminiumhydrid in einer inerten Atmosphäre und gewünschtenfalls
die Schicht (C) durch Gasphasenzersetzung flüchtiger Metall- oder Phosphorverbindungen in Gegenwart von Sauerstoff und/oder Wasserdampf
auf die metallischen Substratplättchen aufbringt.

Schließlich wurde die Verwendung dieser Glanzpigmente und Glanzpigmentmischungen zur Einfärbung von Lacken, Druckfarben, Tinten, Kunststoffen, Gläsern, keramischen Produkten und Zubereitungen der dekorativen Kosmetik gefunden.

Für die erfindungsgemäßen Glanzpigmente sind als Substrat alle für Metalleffektpigmente bekannten Metalle und Legierungen in Plättchenform geeignet. Z.B. kommen neben Stahl, Kupfer und seinen Legierungen wie Messing und Bronzen vor allem Aluminium und seine Legierungen wie Aluminiumbronze in Betracht.

Bevorzugt sind Aluminiumflakes, die in einfacher Weise durch Herausstanzen aus Aluminiumfolie oder nach gängigen Verdüsungs-und Mahltechniken herzustellen sind.

So sind beispielsweise Aluminiumplättchen geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschrieben, durch oxidative Behandlung entfernt werden.

Weiterhin können die metallischen Substratteilchen passiviert sein, d.h. insbesondere gegenüber Wasser stabilisierende Beschichtungen aufweisen, wie sie z.B. aus der DE-A-42 36 332 und der nicht vorveröffentlichten DE-A-44 14 079 bekannt sind.

Als passivierende Beschichtungen sollen dabei auch Metalloxidschichten verstanden werden. Beispiele für weitere geeignete Substrate sind daher eisenoxidbeschichtete Metallpigmente (z.B. EP-A-33 457) mit (schwacher) goldener bis roter Eigenfarbe und zart pastellfarbene titandioxidbeschichtete Metallpigmente (z.B. EP-A-338 428). Die Metalloxidschicht sollte jedoch nicht zu dick sein, damit die Substratteilchen ihre "metallische Koloristik" behalten.

Schließlich sind auch magnetisierbare Aluminiumplättchen als Substratmaterial geeignet, die eine ferromagnetische, Eisen, Cobalt, Nickel, Magnetit oder γ-Fe₂O₃ enthaltende Beschichtung aufweisen (DE-A-43 13 541 und die nicht vorveröffentlichten DE-A-43 40 141 und 44 19 173) und die Herstellung magnetisierbarer goniochromatischer Glanzpigmente ermöglichen.

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von etwa 0,1 bis 5 µm, insbesondere um etwa 0,5 µm. Ihre spezifische freie Oberfläche (BET) beträgt im allgemeinen 0,1 bis 5 m²/g.

Die erfindungsgemäßen Glanzpigmente zeichnen sich durch eine Mehrfachbeschichtung des metallischen Substrats aus.

Die Schicht (A) enthält als wesentliche Bestandteile Aluminiumoxid, Aluminiumoxidhydrat und bevorzugt Siliciumoxid und Siliciumoxidhydrat sowie auch deren Mischungen.

Die Dicke der Schicht (A) beträgt im allgemeinen 20 bis 800 nm, bevorzugt 50 bis 600 nm. Da die Schicht (A) im wesentlichen den Farbton der erfindungsgemäßen Pigmente bestimmt, hat sie für ein besonders ausgeprägtes Farbenspiel zeigende und daher auch bevorzugte Glanzpigmente, die nur ein Schichtpaket (A) + (B) aufweisen, eine Mindestschichtdicke von etwa 100 nm.

Mit wachsender Schichtdicke von (A) durchläuft man bei den mit der Schicht (A) und der metallischen Schicht (B) belegten Pigmenten bei einem Betrachtungswinkel von 25° mehrmals nacheinander die Interferenzfarben blau, grün, gold, rot. Die Winkelabhängigkeit des Farbtons nimmt von der ersten Interferenzfarbenserie nach höheren Serien (also dicker werdenden Schichten (A)) zu. So kippt beispielsweise ein rötlicher Goldton der ersten Serie winkelabhängig ab in ein grünliches Gold, während ein solcher Farbton aus der zweiten oder dritten Interferenzserie in die Komplementärfarbe, ein grünstichiges Blau, umschlägt.

Die im wesentlichen aus metallischem Aluminium bestehende Schicht (B) sollte für sichtbares Licht zumindest teilweise durchlässig (semitransparent) sein und hat daher in der Regel eine Schichtdicke von 1 bis 25 nm, vorzugsweise 5 bis 20 nm.

Sind mehrere (z.B. 2, 3 oder 4) Schichtpakete (A) + (B) enthalten, dann ist die Schicht (A) insbesondere 20 bis 400 nm dick und die Schicht (B) bevorzugt 2 bis 5 nm dick. Bevorzugt sind jedoch die Glanzpigmente mit nur einem Schichtpaket (A) + (B).

Die erfindungsgemäßen Glanzpigmente können noch eine äußere Schicht (C) aufweisen, die insbesondere zum Schutz der darunterliegenden Aluminiumschicht (B) dient.

Die Schicht (C) kann aus niedrigbrechenden oder hochbrechenden Metalloxiden, die sowohl farblos als auch selektiv absorbierend sein können, aufgebaut sein. Geeignet sind z.B. Siliciumoxid, Siliciumoxidhydrat, Aluminiumoxid, Aluminiumoxidhydrat, Zinnoxid, Titandioxid, Zirkonoxid, Eisen(III)oxid und Chrom(III)oxid, wobei Siliciumoxid und vor allem Aluminiumoxid, das vorteilhaft durch Anoxidation der Aluminiumoberfläche der Schicht (B) gebildet werden kann, bevorzugt sind.

Die Schicht (C) kann jedoch auch eine durch Gasphasenpassivierung zu erhaltende, phosphat-, chromat- und/oder vanadathaltige oder auch phosphat- und SiO₂-haltige Schicht sein (DE-A-42 36 332 und die nicht vorveröffentlichte DE-A-44 14 079), die insbesondere auch den Einsatz der erfindungsgemäßen Glanzpigmente in Wasserbasislacken oder anderen wäßrigen Systemen ermöglicht.

Die Dicke der Schicht (C) beträgt im allgemeinen etwa 1 bis 400 nm, vorzugsweise 5 bis 250 nm. Dabei sind z.B. für SiO₂-Schichten (C) Schichtdicken von 50 bis 250 nm und für durch Anoxidation der Aluminiumschicht (B) entstandene Al₂O₃-Schichten (C) und phosphat- und gegebenenfalls SiO₂-haltige Passivierungsschichten (C) Schichtdicken von 5 bis 20 nm bevorzugt.

Selbstverständlich kann auch die Schicht (C) zur Interferenz des Pigmentes beitragen und dabei die Interferenzenreihe an der durch das mit (A) und (B) beschichtete Substrat bestimmten Stelle fortsetzen. Dies ist beispielsweise der Fall, wenn Zirkon- oder Titanoxid als Schicht (C) aufgebracht werden. Besteht dagegen die Schicht (C) im wesentlichen aus Siliciumoxid, so wird sich diese Schicht im Anwendungsmedium (z.B. Lacken, Druckfarben oder Tinten), das einen ähnlichen Brechungsindex aufweist, koloristisch kaum bemerkbar machen.

Farbige Metalloxide wie Eisen- und Chromoxid werden die Interferenzfarbe des Mehrschichtsystems durch Beimischen ihrer Absorptionsfarbe modifizieren und mit zunehmender Schichtdicke schließlich überdecken.

Insgesamt zeichnen sich bei den erfindungsgemäßen Glanzpigmenten alle Schichten durch ihren gleichmäßigen, homogenen und filmartigen Aufbau und ihre Fähigkeit zur Interferenz auch der dickeren Schichten aus, so daß kräftige Interferenzfarben zeigende Mehrschichtsysteme entstehen, bei denen die Substratteilchen an allen Seiten und nicht nur an der Plättchenober- und -unterseite beschichtet sind.

Aus koloristischen Gründen sind auch Mischungen der erfindungsgemäßen metallischen Pigmente (I) mit ebenfalls mehrfach beschichteten silikatischen Plättchen (II) von besonderem Interesse.

Als silikatische Substrate kommen dabei insbesondere helle bzw. weiße Glimmer in Betracht, wobei Schuppen von vorzugsweise naß vermahlenem Muskovit besonders bevorzugt sind. Selbstverständlich sind auch andere natürliche Glimmer wie Phlogopit und Biotit, künstliche Glimmer, Talk- und Glasschuppen geeignet.

Die zum Einsatz kommenden silikatischen Substratteilchen weisen eine Metalloxidschicht (A') auf, die vorzugsweise aus hochbrechenden Metalloxiden wie Titan-, Zirkon-, Zink-, Zinn-, Chrom-, Eisenoxid und/oder Bismutoxychlorid aufgebaut ist. Aluminium- und Siliciumoxid können ebenfalls enthalten sein.

Besonders bevorzugt sind Glimmerpigmente, die eine im wesentlichen aus Titandioxid bestehende und die weiteren genannten Oxide höchstens in untergeordneter Menge enthaltende Schicht (A') aufweisen.

Metalloxidbeschichtete silikatische Pigmente sind allgemein bekannt und auch unter den Bezeichnungen Iriodin^{®} (Merck, Darmstadt), Flonac^{®} (Kemira Oy, Pori) oder Mearlin^{®} (Mearl Corporation, New York) im Handel.

Durch geeignete Wahl der silikatischen Pigmente (II) kann das Farbenspiel der Metallpigmente (I) variiert oder ergänzt werden.

Zeigt beispielsweise ein mit (A) und (B) beschichtetes metallisches Substrat bei einem Betrachtungswinkel von 25° einen goldenen Farbton, so kann dieser durch Beimischen eines eine rotgoldene Interferenzfarbe aufweisenden titandioxidbeschichteten Glimmerpigments zu dem nur mit (A) beschichteten Metallpigment und anschließende gemeinsame Beschichtung mit (B) in Richtung auf einen rötlicheren Farbton verschoben werden.

Die Zusammensetzung der erfindungsgemäßen Glanzpigmentmischungen wird von der gewünschten Koloristik bestimmt.

Prinzipiell kann das Gewichtsverhältnis metallisches Pigment (I): silikatisches Pigment (II) von 1:99 bis 99:1 variiert werden. Um ein ausreichendes Deckvermögen zu erreichen, enthalten die erfindungsgemäßen Pigmentmischungen vorzugsweise mindestens 5 Gew.-% metallisches Glanzpigment (I).

Der bevorzugte Weg zur Herstellung der erfindungsgemäßen Pigmentmischungen ist die gemeinsame Beschichtung der bereits gemäß Schritt (a) mit der Schicht (A) und mit einer Schicht (A') belegten Substratteilchen mit der metallischen Schicht (B) und gewünschtenfalls der Deckschicht (C).

Selbstverständlich können jedoch auch alle Schichten getrennt aufgebracht werden und die beschichteten Pigmente dann anschließend gemischt werden. Bei dieser Vorgehensweise können die Schichten (B) und (B') sowie (C) und (C') zusätzlich variiert werden.

Bei dem erfindungsgemäßen Verfahren zur Herstellung der Glanzpigmente werden die einzelnen Schichten durch Gasphasenzersetzung geeigneter flüchtiger Metallverbindungen (chemical vapor deposition, CVD) oder naßchemisch durch hydrolytische Zersetzung insbesondere organischer Metallverbindungen aufgebracht.

Selbstverständlich können beide Vorgehensweisen beliebig zur Herstellung der einzelnen Schichten kombiniert werden.

Zur Erzeugung der Silicium- und/oder Aluminiumoxidschichten (A) sind die naßchemische und die CVD-Verfahrensvariante gleichermaßen geeignet, jedoch wird meist die CVD-Variante vorzuziehen sein, da die Aluminiumschichten (B) erfindungsgemäß aus der Gasphase abgeschieden werden. Eine Zwischenisolierung und Trocknung des mit (A) belegten Pigments kann dann entfallen.

Bei der in der nicht vorveröffentlichten DE-A-44 05 492 beschriebenen naßchemischen Variante werden organische Silicium- und/oder Aluminiumverbindungen, bei denen die organischen Reste über Sauerstoffatome an die Metalle gebunden sind, in Gegenwart der Substratteilchen und eines organischen Lösungsmittels, in welchem die Metallverbindungen löslich sind, hydrolysiert.

Hierfür sind eine Vielzahl von organischen Lösungsmitteln geeignet, bevorzugt ist Isopropanol.

Bevorzugte Beispiele für die metallischen Ausgangsverbindungen sind die Acetylacetonate und insbesondere Alkoholate, vor allem C₁-C₄-Alkanolate, z.B. Aluminiumtriisopropanolat und Tetraethoxysilan.

Die Hydrolyse wird vorzugsweise in Gegenwart einer Base oder einer Säure als Katalysator durchgeführt. Hierfür eignen sich z.B. neben Alkalilaugen wie Natronlauge insbesondere wäßrige Ammoniaklösungen. Geeignete saure Katalysatoren sind beispielsweise Phosphorsäure und organische Säuren wie Essigsäure und Oxalsäure.

Wasser muß mindestens in der stöchiometrisch für die Hydrolyse erforderlichen Menge vorliegen, bevorzugt ist jedoch die 2 bis 100fache, insbesondere die 5 bis 20fache Menge.

Bezogen auf die eingesetzte Wassermenge, gibt man in der Regel 3 bis 40 Vol.-%, vorzugsweise 5 bis 30 Vol.-%, einer 25 gew.-%igen wäßrigen Ammoniaklösung zu.

Für die Temperaturführung hat es sich als vorteilhaft erwiesen, das Reaktionsgemisch innerhalb von 10 bis 48 h schrittweise auf Rückflußtemperatur zu erhitzen. Bei Verwendung von Isopropanol als Lösungsmittel rührt man das Gemisch zum Beispiel bevorzugt zunächst 4 bis 20 h bei 40°C, dann 4 bis 20 h bei 60°C und zum Schluß 2 bis 8 h bei 80°C.

Verfahrenstechnisch geht man bei Schritt a) des erfindungsgemäßen Herstellungsverfahrens zweckmäßigerweise wie folgt vor:

Man legt Substratteilchen, organisches Lösungsmittel, Wasser und Katalysator (Säure oder bevorzugt Base, insbesondere z.B. eine wäßrige Ammoniaklösung) vor und gibt die zu hydrolysierende Metallverbindung, pur oder gelöst, z.B. als 30 bis 70, bevorzugt 40 bis 60 vol.-%ige Lösung im organischen Lösungsmittel, zu. Erfolgt die Zugabe der Metallverbindung in einem Schritt, dann wird die Suspension anschließend wie oben beschrieben unter Rühren erhitzt. Man kann die Metallverbindung aber auch bei erhöhter Temperatur kontinuierlich zudosieren, wobei Wasser und Ammoniak vorgelegt oder ebenfalls kontinuierlich zudosiert werden können. Nach beendeter Beschichtung wird die Reaktionsmischung wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gewünschtenfalls kann man den Beschichtungsschritt ein- oder mehrfach wiederholen.

Bei der in der nicht vorveröffentlichten DE-A-44 37 752 beschriebenen CVD-Variante werden Silane, die mindestens einen Alkanoyloxyrest enthalten, in der Gasphase mit Wasserdampf und gegebenenfalls Sauerstoff in Gegenwart bewegter Substratteilchen zersetzt.

Hierfür geeignete Silane entsprechen insbesondere der Formel

RₐSiX_{b}Y_{c}

in der die Variablen folgende Bedeutung haben:
- R: Alkyl, bevorzugt C₁-C₁₀-Alkyl, besonders bevorzugt C₁-C₆-Alkyl, das durch Chlor substituiert sein kann, ein- oder mehrfach ungesättigt sein kann und dessen Kohlenstoffkette durch eine oder mehrere Iminogruppen oder Sauerstoffatome in Etherfunktion unterbrochen sein kann; Phenyl, das durch C₁-C₂-Alkyl substituiert sein kann, oder Wasserstoff;
- X: Alkoxy, bevorzugt C₁-C₆-Alkoxy, besonders bevorzugt C₄-Alkoxy, vor allem tert.-Butoxy;
- Y: Alkanoyloxy, bevorzugt C₂-C₃-Alkanoyloxy, besonders bevorzugt Acetoxy;

- a: 0 bis 3, bevorzugt 0 bis 2, besonders bevorzugt 0;
- b: 0 bis 3, bevorzugt 1 bis 3, besonders bevorzugt 2;
- c: 1 bis 4, bevorzugt 1 bis 3, besonders bevorzugt 2,
wobei die Summe a+b+c=4 ist und die Reste R für a>1, die Reste X für b>1 und die Reste Y für c>1 jeweils gleich oder verschieden sein können.

Besonders geeignet sind die Silane, die bei Temperaturen ≤ 600°C, aus technischen Gründen insbesondere auch ≤ 300°C, einen ausreichend hohen Dampfdruck aufweisen, um eine einfache Verdampfung zu gewährleisten, und auch leicht mit Wasserdampf und/oder Luft zersetzt und als Oxid abgeschieden werden können. Selbstverständlich können auch Gemische verschiedener Silane eingesetzt werden.

Im einzelnen seien beispielhaft folgende bevorzugte Silane genannt:

Tetraacetoxysilan, Methoxy-, Ethoxy-, Propoxy-, Isopropoxy-, Butoxy-, Isobutoxy-, sec.-Butoxy- und tert.-Butoxytriacetoxysilan, Dimethoxy-, Diethoxy-, Dipropoxy-, Diisopropoxy-, Dibutoxy-, Diisobutoxy-, Di-sec.-butoxy- und Di-tert.-butoxydiacetoxysilan und Trimethoxy-, Triethoxy-, Tripropoxy-, Triisopropoxy-, Tributoxy-, Triisobutoxy-, Tri-sec.-butoxyund Tri-tert.-butoxyacetoxysilan sowie auch Silane, die verschiedene Alkoxyreste enthalten, z.B. Methoxyethoxydiacetoxysilan.

Ganz besonders bevorzugt ist Di-tert.-butoxydiacetoxysilan.

Zur Durchführung der CVD-Variante empfiehlt sich wie allgemein für CVD-Verfahren die Verwendung eines Wirbelschichtreaktors, wie er beispielsweise in der EP-A 45 851 beschrieben ist. Die Substratteilchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (sowie gegebenenfalls Sauerstoff) werden dann mit Hilfe inerter Trägergasströme (vorteilhaft Teilströmen des Wirbelgases) aus vorgeschalteten Verdampfergefäßen über getrennte Düsen eingeleitet.

Um homogene, die Substratteilchen vollständig umhüllende, filmartige Siliciumoxidschichten zu erhalten, wird die Silankonzentration zweckmäßigerweise bei ≤ 5 Vol.-%, vorzugsweise ≤ 2 Vol.-%, bezogen auf die Gesamtgasmenge im Reaktor, gehalten.

Die zur Zersetzung erforderliche Menge Wasserdampf hängt von der Konzentration des Silans ab und sollte mindestens der stöchiometrisch zur Hydrolyse erforderlichen Menge entsprechen, bevorzugt ist jedoch die 10 bis 100fache Menge.

Enthält das Silan Alkyl- oder Phenylsubstituenten R, so empfiehlt sich die Anwesenheit von Sauerstoff bei der Zersetzung, wenn Kohlenstoffreste, die sich in der Regel bei der alleinigen Verwendung von Wasserdampf bilden, in der abgeschiedenen Siliciumoxidschicht vermieden werden sollen.

Die Aluminiumschichten (B) werden beim erfindungsgemäßen Herstellungsverfahren durch Gasphasenzersetzung von Aluminiumorganylen, insbesondere Aluminiumalkylen, oder Alkylaminaddukten von Aluminiumhydriden auf die mit (A) belegten Substratteilchen aufgebracht.

Besonders geeignet sind hierfür flüchtige Aluminiumalkyle der Formel

RₐAlX_{b}

in der R C₁-C₄-Alkyl (Methyl, Ethyl, Propyl, Isopropyl, Butyl, Isobutyl oder tert.-Butyl), X Wasserstoff oder Halogen (Fluor, Chlor, Brom oder Iod), a 1 bis 3 und b 0 bis 2 bedeutet, wobei die Reste R für a >1 gleich oder verschieden sein können, was auch für die Reste X gilt, wenn b=2 ist.

Neben Monoalkylaluminiumhydriden und -halogeniden RAlX₂ eignen sich vorzugsweise Dialkylaluminiumhydride und -halogenide R₂AlX wie Dimethylaluminiumhydrid, Diethylaluminiumhydrid und Diisobutylaluminiumhydrid und insbesondere Aluminiumtrialkyle wie Tripropylaluminium, Tributylaluminium, Triisobutylaluminium, Tri-tert.-butylaluminium und vor allem Triethylaluminium und Trimethylaluminium, die auch in Form von Mischungen zum Einsatz kommen können.

Anstelle der Aluminiumalkyle kann man Tri(C₁-C₄-alkyl)aminaddukte von Aluminiumhydrid, insbesondere die Mono- und die Diaddukte verwenden, beispielsweise AlH₃ N(CH₃)₃, AlH₃ · N(C₂H₅)₃, AlH₃ 2N(CH₃)₃, AlH₃ 2N(C₂H₅)₃ und AlH₂(BH₄) 2N(CH₃)₃.

Verfahrenstechnisch geht man beim Aufbringen der Aluminiumschichten (B) zweckmäßigerweise so vor, daß man das Aluminiumalkyl in einem dem Beschichtungsreaktor vorgeschalteten, schrittweise auf ca. 100 bis 150°C erwärmten Verdampfergefäß als Lösung in einem schwerflüchtigen Kohlenwasserstoff wie Petroleum vorlegt, mit Hilfe eines durch diese Lösung geleiteten Inertgasstroms (z.B. Argon oder insbesondere Stickstoff) über eine vorzugsweise temperierte Düse in den Reaktor überführt und dort bei in der Regel 100 bis 500°C, vorzugsweise 150 bis 400°C, thermisch zersetzt, ) wobei sich auf den im Reaktor bewegten, mit der Schicht (A) belegten Substraten ein Aluminiumfilm abscheidet. Die Gasmenge der flüchtigen Aluminiumverbindung sollte hierbei im allgemeinen nicht mehr als 2 Vol.-%, bevorzugt nicht mehr als 1 Vol.-%, der Gesamtgasmenge im Reaktor betragen.

Als Reaktor wird insbesondere der oben erwähnte Wirbelschichtreaktor bevorzugt, man kann jedoch auch einen Einhalsrundkolben aus Quarzglas verwenden, der über einen Motor gedreht wird, mit Gaszu- und -ableitungen in der Drehachse versehen ist und von einem zweischaligen Klappofen beheizt wird.

Im Prinzip läßt sich jeder beheizbare Mischer, der die Substratteilchen mittels entsprechender Einbauten schonend bewegt und eine Gaszu- und -ableitung gestattet, als Reaktor einsetzen.

Für eine kontinuierliche Verfahrensführung im technischen Maßstab eignet sich z.B. auch ein Drehrohrofen, dem die Substratteilchen und die Aluminiumalkyl/Inertgas-Mischung fortlaufend zugeführt werden.

Aus Metalloxiden aufgebaute äußere Schichten (C) werden beim erfindungsgemäßen Verfahren durch hinlänglich bekannte oxidative Gasphasenzersetzung der Metallcarbonyle (z.B. Eisenpentacarbonyl, Chromhexacarbonyl) bzw. hydrolytische Gasphasenzersetzung der Metallalkoholate (z.B. Titan- und Zirkon-tetra-n- und -isopropanolat) (EP-A-33 457, EP-A-338 428) oder durch die oben beschriebene Gasphasenhydrolyse von Siliciumorganylen aufgebracht.

Al₂O₃-Schichten (C) können vorteilhaft durch kontrollierte Oxidation beim sonst unter Inertgas erfolgenden Abkühlen der mit Aluminium (B) beschichteten Pigmente erhalten werden.

Dabei geht man zweckmäßigerweise so vor, daß man beim Abkühlen, beginnend bei etwa 80 bis 120°C, portionsweise Luft zudosiert und die Reaktortemperatur beobachtet. Wenn dickere Al₂O₃-Schichten (≥5 nm) gebildet werden sollen, empfiehlt es sich, nach der ersten Anoxidation bei Temperaturen von etwa 100°C bis etwa 400°C weitere Luft, die vorzugsweise mit Wasserdampf gesättigt ist, zuzuführen.

Hierbei ist zu berücksichtigen, daß die Al₂O₃-Schicht auf Kosten des abgeschiedenen Aluminiumfilms wächst und daher von vornherein dickere Aluminiumschichten aufgebracht werden müssen als im Endprodukt enthalten sein sollen.

Phosphat-, chromat- und/oder vanadathaltige sowie phosphatund SiO₂-haltige äußere Schichten (C) können nach den in der DE-A-42 36 332 und in der nicht vorveröffentlichten DE-A-44 14 079 beschriebenen Passivierungsverfahren durch hydrolytische oder oxidative Gasphasenzersetzung von Oxidhalogeniden der Metalle (z.B. CrO₂Cl₂, VOCl₃), insbesondere von Phosphoroxyhalogeniden (z.B. POCl₃), Phosphor- und Phosphorigsäureestern (z.B. Di- und Trimethyl- und -ethylphosphit) und von Aminogruppen enthaltenden Siliciumorganylen (z.B. 3-Aminopropyltriethoxy- und -trimethoxysilan) aufgebracht werden.

Glanzpigmente, die in wäßrigen Systemen besonders stabil sind, werden bei kombinierter Zersetzung der Phosphor- und Siliciumverbindungen erhalten.

Mit Hilfe des erfindungsgemäßen Herstellungsverfahrens können die mehrfach beschichteten Glanzpigmente in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäßen Glanzpigmente und Glanzpigmentmischungen eignen sich vorteilhaft für viele Zwecke, wie zur Einfärbung von Kunststoffen, Gläsern, keramischen Produkten, Zubereitungen der dekorativen Kosmetik und insbesondere von Lacken, insbesondere auch Automobillacken, Tinten und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen transparenten, bunten und schwarzen Glanzpigmenten auf der Basis von metalloxidbeschichteten Glimmer- und Metallpigmenten, plättchenförmigen Eisenoxiden, Graphit, Molybdänsulfid und plättchenförmigen organischen Pigmenten verwenden.

### Beispiele

### Herstellung und Anwendung von erfindungsgemäßen Glanzpigmenten

Bei der Einarbeitung der Pigmente in Lack wurden jeweils 0,4 g Pigment in 3,6 g eines Polyester-Mischlackes mit 21 Gew.-% Feststoffanteil eingerührt und 2 min im Red Devil dispergiert. Mit einer Rakel (160 µm Naßfilmdicke) wurden auf schwarzweißem Karton Abzüge der pigmentierten Lacke angefertigt.

Bei der Anwendung der Pigmente im Siebdruck wurden 10 g Pigment in 90 g einer handelsüblichen Bindemittellösung (22,5 g PVC-Mischpolymerisat Laroflex^{®} MP45, 4,5 g Methoxypropylacetat, 13,5 g n-Hexyldiglykol, 49,5 g Butylglykol) eingerührt. Die so hergestellte Siebdruckfarbe wurde mit einer handelsüblichen Siebdruckmaschine (Siebmaschenweite 112 bis 150 µm) auf gestrichenes, titandioxidbeschichtetes Papier in einer Schichtdicke von 45 µm aufgebracht und an der Luft getrocknet.

### Beispiel 1

a) In einem mit Rückflußkühler und Rührapparatur versehenen Rundkolben wurden 100 g feinteiliges Aluminiumpulver (mittlerer Teilchendurchmesser 20 µm) in 1,5 l Isopropanol aufgeschlämmt. Nach Zugabe von 400 ml Wasser und 40 ml einer 25 gew.-%igen wäßrigen Ammoniaklösung wurde die Suspension unter kräftigem Rühren auf 65°C erhitzt. Gleichzeitig wurde mit der Zudosierung eines Gemisches von 600 ml Isopropanol und 600 g Tetraethoxysilan begonnen (Dosiergeschwindigkeit 100 ml/h, 12 h). Nach einer Nachrührzeit von 10 h und Abkühlen der Suspension wurde das Produkt abfiltriert, gründlich mit Isopropanol gewaschen und bei 80°C getrocknet.
   Das beschichtete Aluminiumpulver hatte einen SiO₂-Gehalt von 59,3 Gew.-% und zeigte einen schwachen Grünstich.
b) Anschließend wurden 200 g des beschichteten Aluminiumpulvers im Wirbelschichtreaktor (beschrieben in der EP-A-571 836) unter Fluidisierung mit insgesamt 800 l/h Stickstoff auf 200°C erhitzt. Aus einer Verdampfervorlage, die unter Argon mit 63,5 g einer 20 gew.-%igen Lösung von Triethylaluminium in Petroleum (Siedebereich 175-255°C) befüllt, 2 h auf 50°C gehalten und dann innerhalb mehrerer Stunden schrittweise auf 120°C erhitzt worden war, wurde das Triethylaluminium mit einem Argonstrom von 100 l/h über eine auf die Verdampfertemperatur abgestimmte, temperierte Düse in insgesamt etwa 8 h in den Reaktor überführt und dort zu Aluminium, Ethylen und Wasserstoff zersetzt. Anschließend wurde der Reaktor noch 2 h bei 200°C gehalten. Beim Abkühlen auf Raumtemperatur wurde mehrmals in kleinen Portionen (ca. 500 ml) Luft eingeleitet und so die Oberfläche des abgeschiedenen Aluminiumfilms passiviert.

Das erhaltene Pigment hatte einen SiO₂-Gehalt von 58,4 Gew.-% und einen Aluminiumgehalt von insgesamt 41,6 Gew.-%, was einem Anteil der Aluminiumbeschichtung am Pigment von 1,5 Gew.-% entspricht.

Das Pigment zeigte bei Applikation im Lack und im Siebdruck bei starkem metallischen Glanz eine intensiv grüne Interferenzfarbe, die bei steileren Betrachtungswinkeln in ein rotstichiges Blau abkippte. Eine mit einem kommerziellen Farbkopierer (Canon CLC 500) hergestellte Farbkopie des Siebdrucks zeigte kein winkelabhängiges Farbenspiel mehr, sondern lediglich eine Mischfarbe.

### Beispiel 2

Es wurde analog zu Beispiel 1 vorgegangen, jedoch wurde in Schritt b) die vierfache Menge Triethylaluminium zersetzt. Aufgrund der hohen Schichtdicke der aufgebrachten Aluminiumschicht zeigte das Pigment nur noch kaum wahrnehmbare Interferenzfarben. Beim Abkühlen wurde die Aluminiumoberfläche wie in Beispiel 1 mit Luft passiviert.

Anschließend wurde den Wirbelgasen ein mit Wasserdampf gesättigter Luftstrom zugesetzt, und der Reaktor wurde schrittweise auf 100°C erhitzt. Nach 5 h war das Pigment durch Anoxidation der Aluminiumschicht wie in Beispiel 1 intensiv grün gefärbt.

### Beispiel 3

Es wurde analog zu Beispiel 1 vorgegangen, jedoch wurde in Schritt b) die doppelte Menge Triethylaluminium zersetzt.

Nach beendeter Aluminiumabscheidung wurde die Reaktortemperatur auf 190°C gesenkt. Dann wurde ein Teil der Wirbelgase (200 l/h) über eine auf 40°C temperierte Vorlage mit 25 ml Phosphoroxychlorid geleitet. Mit weiteren 200 l/h Stickstoff wurde über eine auf 50°C temperierte Wasservorlage zusätzlich Wasserdampf in den Reaktor überführt. Nach Beendigung der POCl₃-Zufuhr (4 h) wurde die Verdampfervorlage mit 20 ml 3-Aminopropyltriethoxysilan befüllt und auf 110°C erwärmt, die Reaktortemperatur wurde auf 220°C erhöht. Nach 5 h war das Silan vollständig verdampft.

Das zweifach passivierte Pigment zeigte eine nahezu unverändert intensive grüne Interferenzfarbe wie in Beispiel 1.

## Patentansprüche

1. Goniochromatische Glanzpigmente auf der Basis von durch Mehrfachbeschichtung vollständig umhüllten plättchenförmigen metallischen Substraten, die mindestens ein Schichtpaket aus
A) einer im wesentlichen aus Siliciumoxid, Siliciumoxidhydrat, Aluminiumoxid und/oder Aluminiumoxidhydrat bestehenden Schicht und
B) einer im wesentlichen aus Aluminium bestehenden, metallischen, für sichtbares Licht zumindest teilweise durchlässigen Schicht
sowie gewünschtenfalls zusätzlich
C) eine äußere Schicht, die im wesentlichen aus farblosem oder selektiv absorbierendem Metalloxid besteht und/oder phosphat-, chromat- und/oder vanadathaltig ist,
aufweisen.

2. Glanzpigmente nach Anspruch 1, bei denen die Schicht (C) als Metalloxid Siliciumoxid, Siliciumoxidhydrat, Aluminiumoxid, Aluminiumoxidhydrat, Titandioxid, Zirkondioxid, Eisen(III)-oxid und/oder Chrom(III)oxid enthält.

3. Glanzpigmente nach Anspruch 1 oder 2, die nur ein Schichtpaket (A) + (B) enthalten.

4. Glanzpigmente nach den Ansprüchen 1 bis 3, bei denen das metallische Substrat im wesentlichen aus Aluminiumplättchen besteht, die nach gängigen Verdüsungs- und Mahltechniken hergestellt worden sind.

5. Glanzpigmente nach den Ansprüchen 1 bis 4, bei denen das metallische Substrat im wesentlichen aus Aluminiumplättchen, die mit einer ferromagnetischen Schicht belegt und/oder passiviert sein können, besteht.

6. Glanzpigmentmischungen aus
I) den Glanzpigmenten gemäß den Ansprüchen 1 bis 5 und
II) mehrfach beschichteten silikatischen Plättchen, die
A') eine im wesentlichen aus farblosem oder selektiv absorbierendem Metalloxid bestehende Schicht,
B') eine im wesentlichen aus Aluminium bestehende, metallische, für sichtbares Licht zumindest teilweise durchlässige Schicht und gewünschtenfalls
C') eine äußere Schicht, die im wesentlichen aus farblosem oder selektiv absorbierendem Metalloxid besteht und/oder phosphat-, chromat- und/oder vanadathaltig ist,
aufweisen,
als wesentlichen Komponenten.

7. Verfahren zur Herstellung von Glanzpigmenten gemäß den Ansprüchen 1 bis 5, dadurch gekennzeichnet, daß man
die Schichten (A) durch Gasphasenzersetzung flüchtiger Siliciumorganyle mit Wasserdampf und/oder Sauerstoff oder durch hydrolytische Zersetzung organischer Silicium- oder Aluminiumverbindungen, bei denen die organischen Reste über Sauerstoffatome an das Metall gebunden sind, in Gegenwart eines organischen Lösungsmittels, in welchem die Metallverbindungen löslich sind, und anschließende Trocknung,
die metallischen Schichten (B) durch Gasphasenzersetzung flüchtiger Aluminiumalkyle oder Trialkylaminaddukte von Aluminiumhydrid in einer inerten Atmosphäre und gewünschtenfalls
die Schicht (C) durch Gasphasenzersetzung flüchtiger Metalloder Phosphorverbindungen in Gegenwart von Sauerstoff und/ oder Wasserdampf
auf die metallischen Substratplättchen aufbringt.

8. Verfahren zur Herstellung von aluminiumbeschichteten Pigmentplättchen, dadurch gekennzeichnet, daß man verdampfte Aluminiumalkyle oder Trialkylaminaddukte von Aluminiumhydrid in einer inerten Atmosphäre in Gegenwart der Pigmentplättchen zersetzt.

9. Verfahren nach Anspruch 8, dadurch gekennzeichnet, daß man Aluminiumalkyle der allgemeinen Formel
RₐAlX_{b}
in der R gleiche oder verschiedene C₁-C₄-Alkylreste, X Wasserstoff oder Halogen, a 1 bis 3 und b 0 bis 2 bedeutet, oder Tri(C₁-C₄-alkyl)aminaddukte von Aluminiumhydrid zersetzt.

10. Verwendung von Glanzpigmenten gemäß den Ansprüchen 1 bis 6 zur Einfärbung von Lacken, Druckfarben, Tinten, Kunststoffen, Gläsern, keramischen Produkten und Zubereitungen der dekorativen Kosmetik.

## Claims

1. Goniochromatic luster pigments based on multiply and completely coated platelet-shaped metallic substrates comprising at least one layer packet of
A) a layer consisting essentially of silicon oxide, silicon oxide hydrate, aluminum oxide and/or aluminum oxide hydrate, and
B) a metallic layer which consists essentially of aluminum and is at least partially transparent to visible light,
and also, if desired, additionally
C) an outer layer which consists essentially of colorless or selectively absorbing metal oxide and/or is phosphate-, chromate- and/or vanadate-containing.

2. Luster pigments as claimed in claim 1 wherein the metal oxide of layer (C) is silicon oxide, silicon oxide hydrate, aluminum oxide, aluminum oxide hydrate, titanium dioxide, zirconium dioxide, iron(III) oxide and/or chromium(III) oxide.

3. Luster pigments as claimed in claim 1 or 2 comprising only one layer packet (A) + (B).

4. Luster pigments as claimed in any of claims 1 to 3 wherein the metallic substrate consists essentially of aluminum platelets produced by common atomizing and grinding techniques.

5. Luster pigments as claimed in any of claims 1 to 4 wherein the metallic substrate consists essentially of aluminum platelets coated and/or passivated with a ferromagnetic layer.

6. Luster pigment mixtures of
I) the luster pigments of any of claims 1 to 5, and
II) multiply coated silicatic platelets comprising
A') a layer consisting essentially of colorless or selectively absorbing metal oxide,
B') a metallic layer which consists essentially of aluminum and is at least partially transparent to visible light, and if desired
C') an outer layer which consists essentially of colorless or selectively absorbing metal oxide and/or is phosphate-, chromate- and/or vanadate-containing,
as essential components.

7. A process for producing luster pigments as claimed in any of claims 1 to 5, which comprises coating the metallic substrate platelets with
layers (A) by gas phase decomposition of volatile organosilicons using water vapor and/or oxygen or by hydrolytic decomposition of organic silicon or aluminum compounds in which the organic radicals are attached to the metal via oxygen atoms in the presence of an organic solvent in which the metal compounds are soluble and subsequent drying,
metallic layers (B) by gas phase decomposition of volatile aluminum alkyls or trialkylamine adducts of aluminum hydride in an inert atmosphere and if desired
layer (C) by gas phase decomposition of volatile metal or phosphorus compounds in the presence of oxygen and/or water vapor.

8. A process for producing aluminum-coated pigment platelets, which comprises decomposing vaporized aluminum alkyls or trialkylamine adducts of aluminum hydride in an inert atmosphere in the presence of pigment platelets.

9. A process as claimed in claim 8 wherein aluminum alkyls of the general formula
RₐAlX_{b}
where R is identical or different C₁-C₄-alkyl, X is hydrogen or halogen, a is from 1 to 3, and b is from 0 to 2, or tri(C₁-C₄-alkyl)amine adducts of aluminum hydride are decomposed.

10. The use of luster pigments as claimed in any of claims 1 to 6 for coloring paints, inks, including printing inks, plastics, glasses, ceramic products and decorative cosmetic preparations.

## Revendications

1. Pigments brillants goniochromatiques à base de substrats métalliques sous forme de plaquettes entièrement recouvertes par plusieurs revêtements, qui présentent au moins un ensemble de couches constitué
A)d'une couche formée essentiellement d'oxyde de silicium, d'oxyde de silicium hydraté, d'oxyde d'aluminium et/ou d'oxyde d'aluminium hydraté et
B) d'une couche métallique constituée essentiellement d'aluminium, laissant au moins partiellement passer la lumière visible
ainsi qu'éventuellement, en plus
C)une couche extérieure constituée essentiellement d'oxyde métallique incolore ou à absorption sélective et/ou contenant des phosphates, des chromates et/ou des vanadates.

2. Pigments brillants selon la revendication 1, pour lesquels la couche (C) contient, en tant qu'oxyde métallique, de l'oxyde de silicium, de l'oxyde de silicium hydraté, de l'oxyde d'aluminium, de l'oxyde d'aluminium hydraté, du dioxyde de titane, de dioxyde de zirconium, de l'oxyde de fer(lll) et/ou de l'oxyde de chrome(III).

3. Pigments brillants selon la revendication 1 ou 2, ne contenant que l'ensemble de couches (A) + (B).

4. Pigments brillants selon l'une quelconque des revendications 1 à 3, pour lesquels le substrat métallique est essentiellement constitué de plaquettes en aluminium préparées par des techniques usuelles d'atomisation et de broyage.

5. Pigments brillants selon l'une quelconque des revendications 1 à 4, pour lesquels le substrat métallique est essentiellement constitué de plaquettes en aluminium pouvant être recouvertes d'une couche ferromagnétique et/ou passivées.

6. Mélanges de pigments brillants constitués
I) des pigments brillants selon l'une quelconque des revendications 1 à 5 et
II) de plaquettes silicatées revêtues plusieurs fois, qui présentent
A') une couche constituée essentiellement d'oxyde métallique incolore ou absorbant de façon sélective,
B') une couche métallique essentiellement constituée d'aluminium et laissant au moins partiellement passer la lumière visible et éventuellement,
C') une couche extérieure constituée essentiellement d'oxyde métallique incolore ou à absorption sélective et/ou contenant des phosphates, des chromates et/ou des vanadates,
en tant que composants essentiels.

7. Procédé de préparation de pigments brillants selon l'une quelconque des revendications 1 à 5, caractérisé en ce que l'on dépose sur les plaquettes de substrat métallique
les couches (A) par décomposition en phase gazeuse de composés organiques volatils du silicium à l'aide de vapeur d'eau et/ou d'oxygène ou par décomposition par hydrolyse de composés organiques du silicium ou de l'aluminium, dans lesquels les restes organiques sont liés au métal par des atomes d'oxygène, en présence d'un solvant organique dans lequel les composés métalliques sont solubles, suivie d'un séchage,
les couches métalliques (B) par décomposition en phase gazeuse de composés volatils alkylés de l'aluminium ou d'adduits trialkylaminés d'hydrure d'aluminium dans une atmosphère inerte et éventuellement
la couche (C) par décomposition en phase gazeuse de composés volatils du phosphore ou de métaux en présence d'oxygène et/ou de vapeur d'eau.

8. Procédé de préparation de plaquettes de pigments revêtues d'aluminium, caractérisé en ce que l'on décompose des composés vaporisés alkylés de l'aluminium ou des adduits trialkylaminés d'hydrure d'aluminium dans une atmosphère inerte en présence de plaquettes de pigment.

9. Procédé selon la revendication 8, caractérisé en ce que l'on décompose des composés alkylés de l'aluminium de formule générale
RₐAlX_{b}
dans laquelle R représente des restes alkyle en C₁-C₄ identiques ou différents, X représente un atome d'hydrogène ou d'halogène, a vaut 1 à 3 et b vaut 0 à 2, ou des adduits tri(alkyle en C₁-C₄)aminés d'hydrure d'aluminium.

10. Utilisation de pigments brillants selon l'une quelconque des revendications 1 à 6 pour la coloration de laques, d'encres d'imprimerie, d'encres, de matières plastiques, de verres, de produits céramiques et de préparations pour la cosmétique décorative.
